# EUROPEAN PATENT APPLICATION

(11) **EP 3 960 204 A1**
(43) Date of publication of application: **02.03.2022**
(21) Application number: 20795662.4
(22) Date of filing: 24.04.2020
(51) Int. Cl.: A61K 51/00, A61K 101/00

(54) **BPA LYOPHILIZED PREPARATION AND PREPARATION METHOD THEREFOR**

(30) Priority: 26.04.2019 CN 201910341883
(71) Applicant: Neuboron Medtech Ltd., Nanjing, Jiangsu 211112 (CN)
(72) Inventor: LIU, Yuanhao, Nanjing, Jiangsu 211112 (CN); CHEN, Lamei, Nanjing, Jiangsu 211112 (CN); WU, Xiaoming, Nanjing, Jiangsu 211112 (CN); JI, Hongfeng, Nanjing, Jiangsu 211112 (CN); LI, Yajing, Nanjing, Jiangsu 211112 (CN); HE, Jing, Nanjing, Jiangsu 211112 (CN); WANG, Dongchun, Nanjing, Jiangsu 211112 (CN)
(74) Representative: Gunzelmann, Rainer
(86) International application number: PCT/CN2020/086745
(87) International publication number: WO 2020/216334

(57) **Abstract**

A method for preparing a lyophilized preparation of BPA includes a solution preparation process and a freeze-drying process, where the solution preparation process includes: (1) dissolving BPA and polyol in an aqueous solution by using a base to obtain a clear solution; (2) regulating the clear solution back to 7.5<pH≤8.5 by using an acid, to obtain a BPA solution; and the freeze-drying process includes: (3) subpackaging the BPA solution and freeze-drying under a condition with a vacuum of 10-20 Pa, to obtain the lyophilized preparation. The lyophilized preparation of BPA prepared through the method has good stability and a small content of impurities.

## Description

### BACKGROUND

### Technical Field

The present invention relates to the pharmaceutical field, and specifically, to a lyophilized preparation of BPA and a preparation method.

### Related Art

Boron neutron capture therapy (BNCT) is using boron (¹⁰B)-containing drugs having high capture cross section characteristics for thermal neutrons, to produce two heavy charged particles, ⁴He and ⁷Li, through ¹⁰B(n,α)⁷Li neutron capture and a nuclear fission reaction. The two charged particles have average energy of about 2.33 MeV and characteristics of high linear energy transfer (LET) and a short range. The LET and the range of α particles are 150 keV/µm and 8 µm respectively, while the LET and the range of ⁷Li heavy charged particles are 175 keV/µm and 5 µm respectively. A total range of the two particles is approximately equivalent to a size of a cell, and therefore, radiation damage to organisms can be limited to a cellular level. When the boron-containing drugs are selectively aggregated in tumor cells, the tumor cells can be locally killed by selecting an appropriate neutron source, without causing too much damage to normal tissues.

4-Boronophenylalanine (BPA) is a ¹⁰B-containing drug containing that is studied frequently at present. Because it is difficult to dissolve BPA in water, addition of a solubilizer is generally needed. In addition, the BPA is dissolved under the action of a strong acid or base, and then, the resultant solution is regulated to approximately the physiological pH value, and after being prepared into a sterile injection through sterilization, is applied to patients or animals. The solution needs to be prepared while being used. The cumbersome preparation process causes extremely inconvenient clinical use of the drug, and sterility cannot be guaranteed, which limits application thereof. The Patent CN103100094B discloses a freeze-drying process applicable to L-BPA, where after L-BPA and a solubilizer, fructose, are mixed, the L-BPA is dissolved, and is freeze-dried under vacuum for 22-26 hours.

### SUMMARY

An objective of the present invention is to provide a method for preparing a lyophilized preparation of BPA and a BPA preparation prepared by using the method, where the preparation prepared by using the method has good stability and a small content of impurities.

According to a first aspect, a method for preparing a lyophilized preparation of BPA is provided, including a solution preparation process and a freeze-drying process, where
the solution preparation process includes: (1) dissolving BPA and polyol in an aqueous solution by using a base to obtain a clear solution; (2) regulating the clear solution back to 7.5<pH≤8.5 by using an acid, to obtain a BPA solution; and
the freeze-drying process includes: (3) subpackaging the BPA solution and freeze-drying under a condition with a vacuum of 10-20 Pa, to obtain the lyophilized preparation.

In another preferred example, the temperature of the solution in the solution preparation process is controlled to be lower than or equal to 60°C, preferably, 18 to 50°C, and more preferably, 18 to 40°C.

In another preferred example, a ratio of parts by weight the BPA to the polyol is 1:1-1.3, preferably, 1:1.1-1.25.

In another preferred example, a vacuum of the freeze-drying is 10-20 Pa, preferably, 10-11 Pa.

In another preferred example, the clear solution is regulated back to a pH value of 7.6-8.1, to obtain a BPA solution.

In another preferred example, the base includes: lithium hydroxide, sodium hydroxide, potassium hydroxide, calcium hydroxide, and magnesium hydroxide.

In another preferred example, in the solution preparation process, the BPA and the polyol are dissolved in the aqueous solution by using the base, to obtain a clear solution, and a pH value of the clear solution is 8.5-9.5.

In another preferred example, the polyol includes: fructose, lactose, sorbitol, maltose, mannitol, xylitol, ribose, glucose, and sucrose.

In another preferred example, after the solution preparation process, and before the freeze-drying process, the method further includes a filtering step.

In another preferred example, a time of the freeze-drying process is 39-80 hours.

In another preferred example, a vacuum in the freeze-drying process is 10-11 Pa.

In another preferred example, the freeze-drying process further includes a pre-freezing process. Preferably, the temperature of the pre-freezing process is -20°C to -50°C. Preferably, a time of the pre-freezing process is 5-15 hours.

In another preferred example, the freeze-drying process further includes a sublimation process. Preferably, the temperature of the sublimation process is -20°C to -35°C. Preferably, a vacuum of the sublimation process is 10-20 Pa. Preferably, a time of the sublimation process is 30-55 hours.

In another preferred example, the freeze-drying process further includes a desorption drying process. Preferably, the temperature of the desorption drying process is 20°C to 40°C. Preferably, a vacuum of the desorption drying process is 10-20 Pa. Preferably, a time of the desorption drying process is 4-10 hours.

According to a second aspect, a lyophilized preparation of BPA prepared by using the method according to the first aspect of the present invention is provided.

According to a third aspect, a composition is provided, including the lyophilized preparation of BPA according to the second aspect.

According to a fourth aspect, a kit is provided, including the lyophilized preparation of BPA according to the second aspect.

It should be understood that within the scope of the present invention, the above-mentioned technical features of the present invention and those described in detail in the following (such as embodiments) may be combined with each other, to form new or preferred technical solutions. For the sake of brevity, details are not described herein again.

### BRIEF DESCRIPTION OF THE DRAWINGS

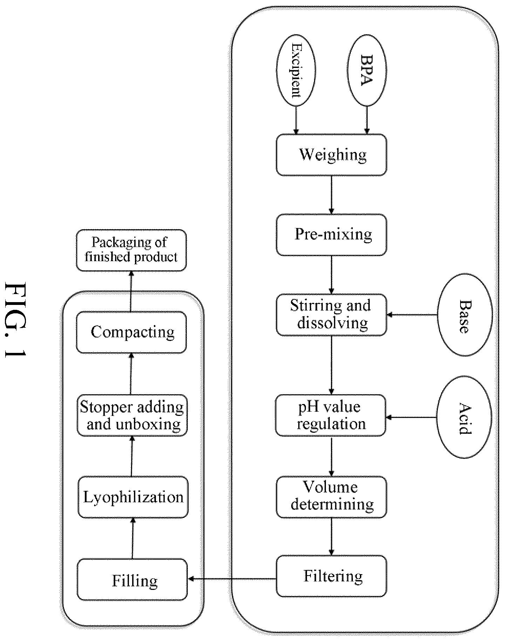

FIG. 1 shows a preferred production process of a lyophilized preparation of BPA according to the present invention.

### DETAILED DESCRIPTION

Unless otherwise defined, all technical and scientific terms in this specification have the same meanings as that usually understood by a person skilled in the art to which the claimed subject belongs. Unless otherwise specified, all patents, patent applications, and publications cited in this specification are incorporated herein by reference in their entirety.

It should be understood that the above brief description and the following detailed description are exemplary and only used for explanation, and are not intended to limit the subject of the present invention. In this application, unless otherwise specified, the plural forms are included when the singular form is used. It should be noted that, unless otherwise clearly specified in this specification, the singular form used in this specification and claims includes the plural referents. It is also noted that, unless otherwise specified, the use of "or", "alternatively" means "and/or". In addition, the terms "comprise", "include", and other grammatical forms such as "comprising" and "including" are not limiting. Section titles in this specification are only used for the purpose of organizing the text, and should not be explained as limitations to the subject. All documents or parts of a document cited in this application, including but not limited to patents, patent applications, articles, books, operating manuals, and papers, are incorporated herein by reference in their entirety.

The "base" described in the present invention is mainly inorganic base, including, but not limited to, lithium hydroxide, sodium hydroxide, potassium hydroxide, calcium hydroxide, and magnesium hydroxide. The "acid" described in the present invention includes inorganic acid and organic acid. The inorganic acid, for example, includes, but is not limited to, perchloric acid, hydroiodic acid, sulfuric acid, hydrobromic acid, hydrochloric acid, nitric acid, iodic acid, oxalic acid (oxalic acid), sulfurous acid, phosphoric acid, pyruvic acid, carbonic acid, citric acid, hydrofluoric acid, malic acid, gluconic acid, formic acid, lactic acid, benzoic acid, acrylic acid, ethylic acid (acetic acid), propionic acid, stearic acid, hydrosulfuric acid, hypochlorous acid, boric acid, trifluoroacetic acid, methanesulfonic acid, benzenesulfonic acid. The preferred acid is hydrochloric acid.

The "BPA" described in the present invention is 4-dihydroxyboryl phenylalanine, of which a chemical formula is 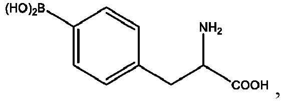 where B includes ¹⁰B and ¹¹B. When being ¹⁰B, B may be used as a drug for the BNCT, and includes two isomers, respectively, L- 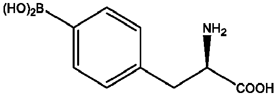 and D- 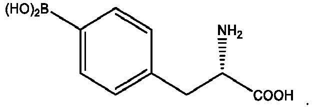 Both configurations should be included in the scope of protection of the present invention, and the BPA of the present invention is preferably L-BPA.

### Method for preparing a lyophilized preparation of BPA

The present invention provides a method for preparing a lyophilized preparation of BPA, including a solution preparation process and a freeze-drying process, where the solution preparation process includes: (1) dissolving BPA and polyol in an aqueous solution by using a base to obtain a clear solution; (2) regulating the clear solution back to 7.5<pH≤8.5 by using an acid, to obtain a BPA solution; and the freeze-drying process includes: (3) subpackaging the BPA solution and freeze-drying under a condition with a vacuum of 10-20 Pa, to obtain the lyophilized preparation. To ensure that chemical properties of components in the solution do not change, in the solution preparation process, the temperature of the solution is controlled to be lower than or equal to 60°C. The "solution" mentioned herein refers to any of the "solutions" in the solution preparation process. Preferably, the temperature is 18 to 50°C, and more preferably, 18 to 40°C.

A ratio of parts by weight of the BPA to polyol affect dissolution of the BPA, but the ratio of parts by weight is not specially limited. Preferably, a ratio of parts by weight the BPA to the polyol is 1:1-1.3, preferably, 1:1.1-1.25. The polyol includes: fructose, lactose, sorbitol, maltose, mannitol, xylitol, ribose, glucose, and sucrose.

In the solution preparation process, the BPA and the polyol are dissolved in the aqueous solution by using the base, to obtain a clear solution, and a pH value of the clear solution is 8.5-9.5. Further, the clear solution needs to be regulated back to a pH value of 7.6-8.1, to obtain a BPA solution.

In the freeze-drying process, a vacuum of the freeze-drying is 10-20 Pa, preferably, 10-11 Pa. A time of the freeze-drying process is 39-80 hours. During actual operation, the above may be determined according to actual parameters in the solution preparation process, and are not specially limited. The freeze-drying process includes: a pre-freezing process, a sublimation process, and a desorption drying process. Parameters, such as a temperature, a time, and a vacuum, in the three processes may be independently selected according to actual needs. In a preferred solution, the BPA solution is freeze-dried according to the following conditions, to obtain a lyophilized preparation of BPA: in a pre-freezing process: reducing the temperature of a sample to -20°C to -60°C, and maintaining for 5-15 hours to completely freeze the sample; in a sublimation process: raising the temperature to -15°C to -35°C, maintaining a vacuum of 10-11 Pa, and maintaining for 30-55 hours; and in a desorption drying process: heating a partition plate to raise the temperature to 0°C to 40°C, maintaining for 4-10 hours, and maintaining a vacuum of 10-11 Pa in the entire desorption drying process. In a preferred solution, in the pre-freezing process, the temperature sometimes may be regulated a plurality of times according to actual needs, for example, is first reduced to -60°C, and maintained for a period of time, and then, is raised to -20 to -50°C. In a preferred solution, in the desorption drying process, the temperature may be first raised to a relatively low temperature, and then be gradually raised to a relatively high temperature, for example, the temperature is first raised to 0°C and maintained for a period of time, and then, is raised to 30±10°C.

### Mainly advantages of the present invention include:

1. In the freeze-drying process, optimized process parameters are used, to greatly prevent incomplete removal of moisture in the freeze-drying process and poor temperature control from exerting adverse impact on the product form, and control the moisture of the product to the lowest level. In addition, the temperature transfer is uniform, the product is puffy and full, and has uniform particles, and phenomena, such as collapse, bubbles, looseness, and shrinkage, may not occur.
2. The product has good resolubility, quick dissolution with water, high purity, and good stability.
3. The product has no visible foreign matter, a low content of related substances, and a good impurity control effect.

### DETAILED DESCRIPTION OF THE EMBODIMENTS

Through wide and thorough researches, the inventor developed a lyophilized preparation of BPA and a preparation method, including a solution preparation process and a freeze-drying process. In the freeze-drying process, optimized process parameters are used, to greatly prevent incomplete removal of moisture in the freeze-drying process and poor temperature control from exerting adverse impact on the product form, so that preparation has good stability and a low content of impurities. The present invention is made on such basis.

The following further describes the present invention with reference to the specific embodiments. It should be understood that the following descriptions are only optimal implementations of the present invention, and should not be regarded as limitations to the protection scope of the present invention. On the basis of a full understanding of the present invention, the experimental methods without specific conditions in the following embodiments are usually in accordance with the conventional conditions or in accordance with the conditions recommended by the manufacturer. A person skilled in the art may make non-essential changes to the technical solutions of the present invention, and such changes should be included in the protection scope of the present invention. Unless otherwise specified, the percentage and the parts are the percentage by weight and the parts by weight respectively.

### Embodiment 1

### 1. Preparation of a BPA solution:

(1) Add 1.0 kg of BPA, 1.1-1.3 kg of fructose, and an appropriate amount of water for injection into a solution preparation tank, wash containers containing the BPA and the fructose with water 3 times, and transfer the water used for washing into the solution preparation tank, and stir for 10 min.
(2) Add a sodium hydroxide solution into the solution preparation tank to dissolve the BPA, and stir until the solution is clear.
(3) Add a hydrochloric acid solution, to regulate a pH value of the solution to 7.6.

Whether the solution after being regulated back and prepared by using the hydrochloric acid is examined. The hydrochloric acid is added into the solution, to regulate the solution to a different pH value close to the physiological pH value. Experiment results indicating whether precipitates are generated in the solution under the condition of pH=7.3-8.5 within 48 hours are observed, as shown in Table 1. In addition, the pH values under the conditions of pH=7.6, 8.0, and 8.5 respectively are shown in Table 2, and the pH value of the solution does not change significantly within 24 hours.

**Table 1 Precipitation statuses of the solution under different pH conditions within 48 hours**

| pH value | Whether the solution precipitates within 48 h |
|---|---|
| 8.5 | No |
| 8.4 | No |
| 8.3 | No |
| 8.2 | No |
| 8.1 | No |
| 8.0 | No |
| 7.9 | No |
| 7.8 | No |
| 7.7 | No |
| 7.6 | No |
| 7.5 | No |
| 7.4 | Yes |
| 7.30 | Yes |

**Table 2 pH value changes of the solution under different pH conditions within 24 hours**

| pH value | Standing time at room temperature | pH value after 24 hours | Characteristic |
|---|---|---|---|
| 7.6 | 0 h | 7.49 | Colorless and clear |
| | 5 h | 7.57 | Colorless and clear |
| | 10 h | 7.63 | Colorless and clear |
| | 15 h | 7.46 | Colorless and clear |
| | 24 h | 7.53 | Colorless and clear |
| 8.0 | 0 h | 8.10 | Colorless and clear |
| | 4 h | 8.10 | Colorless and clear |
| | 16 h | 8.09 | Colorless and clear |
| | 24 h | 8.08 | Colorless and clear |
| 8.5 | 0 h | 8.5 | Colorless and clear |
| | 4 h | 8.5 | Colorless and clear |
| | 8 h | 8.5 | Colorless and clear |
| | 12 h | 8.5 | Colorless and clear |
| | 24 h | 8.5 | Colorless and clear |

### Embodiment 2

The BPA solution is freeze-dried according to the following conditions, to obtain a lyophilized preparation of BPA:
(1) In a pre-freezing process: Reduce the temperature of a sample to -20°C to -60°C, and maintain for 5-15 hours, to completely freeze the sample.
(2) In a sublimation process: Raise the temperature to -15°C to -35°C, maintain a vacuum of 10-11 Pa, and maintain for 30-55 hours.
(3) In a desorption drying process: Heat a partition plate to raise the temperature to 0°C to 40°C, maintain for 4-10 hours, and maintain a vacuum of 10-11 Pa in the entire desorption drying process.

As shown in Table 3, the lyophilized sample is redissolved with water to prepare a solution, the solution stands at room temperature for 24 hours, and none of measurement results of the pH value, the clarity, the transmittance, and the content significantly changes. After the present product is prepared and then, stands at room temperature for 24 hours, the solution has good stability.

**Table 3 Stability of the solution after the lyophilized sample is redissolved**

| Time point (h) | pH | Clarity | Transmittance (%) | Content (%) of impurities |
|---|---|---|---|---|
| 0 | 8.1 | Clear | 99.5 | 0.24 |
| 4 | 8.0 | Clear | 99.4 | - |
| 8 | 8.1 | Clear | 99.4 | 0.26 |
| 12 | 8.0 | Clear | 99.3 | - |
| 24 | 8.0 | Clear | 99.3 | 0.26 |
| 30 | - | Precipitated | - | - |

### Embodiment 3

As shown in FIG. 1, a production process of a lyophilized preparation of BPA includes: ① a preparation procedure; ② a filling procedure; ③ a freeze-drying procedure; ④ an unboxing and capping procedure; and ⑤ a packaging procedure. The process is described in detail as follows:
① Preparation procedure:
   adding water for injection into a solution preparation tank, adding prescription doses of BPA and an excipient, adding a sodium hydroxide solution, washing containers containing the excipient and the sodium hydroxide solution respectively with water for injection, and transferring the water used for washing to the solution preparation tank; stirring until the solution is clear; and regulating the pH value, adding the remaining water for injection, stirring evenly, detecting an intermediate, and filtering, where the excipient is the polyol described in the present invention.
② Filling procedure:
   regulating a filling volume according to a content of the intermediate, filling, partially adding stoppers, and feeding into a freeze-drying box.
③ Freeze-drying procedure:
   including: a pre-freezing stage, a sublimation stage, and a desorption drying stage.
④ Unboxing and capping procedure:
   unboxing: protecting semi-finished products under a 100-level laminar flow, and removing and scrapping an unqualified product that, for example, lacks a stopper, has a displaced stopper, or has a broken bottle; and capping: sampling before, during, and after production to detect appearances of samples.
⑤ Packaging procedure:
   labeling, boxing, encasing, and storing.

All the references mentioned in the present invention are incorporated herein by references, just as if each of the references is individually incorporated by reference. In addition, it should be understood that, after reading the above teaching of the present invention, those skilled in the art can make various changes or modifications to the present invention, and these equivalent forms also fall within the scope defined by the appended claims of this application.

## Claims

1. A method for preparing a lyophilized preparation of BPA, comprising a solution preparation process and a freeze-drying process, wherein
the solution preparation process includes: (1) dissolving BPA and polyol in an aqueous solution by using a base to obtain a clear solution; (2) regulating the clear solution back to 7.5<pH≤8.5 by using an acid, to obtain a BPA solution; and
the freeze-drying process comprises: (3) subpackaging the BPA solution and freeze-drying under a condition with a vacuum of 10-20 Pa, to obtain the lyophilized preparation.

2. The method according to claim 1, wherein the temperature of any one of the "solutions" in the solution preparation process is controlled to be lower than or equal to 60°C.

3. The method according to claim 1, wherein a ratio of parts by weight the BPA to the polyol is 1:1-1.3.

4. The method according to claim 1, wherein the base comprises: lithium hydroxide, sodium hydroxide, potassium hydroxide, calcium hydroxide, and magnesium hydroxide.

5. The method according to claim 1, wherein in the solution preparation process, the BPA and the polyol are dissolved in the aqueous solution by using the base, to obtain a clear solution, and a pH value of the clear solution is 8.5-9.5.

6. The method according to any one of claims 1, 3, and 5, wherein the polyol comprises: fructose, lactose, sorbitol, maltose, mannitol, xylitol, ribose, glucose, and sucrose.

7. The method according to claim 1, wherein after the solution preparation process, and before the freeze-drying process, the method further comprises a filtering step.

8. The method according to claim 1, wherein a time of the freeze-drying process is 39-80 hours.

9. The method according to claim 1, wherein a vacuum of the freeze-drying is 10-11 Pa.

10. The method according to claim 1, wherein the freeze-drying process further comprises:
a pre-freezing process, wherein the temperature of the pre-freezing process is -20°C to -60°C; and/or
a sublimation process, wherein the temperature of the sublimation process is -15°C to - 35°C; and/or
a desorption drying process, wherein the temperature of the desorption drying process is 0°C to 40°C.

11. The method according to claim 1, wherein the freeze-drying process further comprises:
a pre-freezing process, wherein a time of the pre-freezing process is 5-15 hours; and/or
a sublimation process, wherein a time of the sublimation process is 30-55 hours; and/or
a desorption drying process, wherein a time of the desorption drying process is 4-10 hours.

12. The method according to claim 1, wherein a chemical formula of the BPA is: 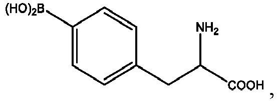 wherein B in the formula is 10B.

13. A lyophilized preparation of BPA prepared by using the method according to claim 1.

14. A composition, comprising the lyophilized preparation of BPA according to claim 13.

15. A kit, comprising the lyophilized preparation of BPA according to claim 13.
